(19) Europäisches Patentamt
European Patent Office
Office européen des brevets

(11) **EP 3 260 041 A2**

(12) **EUROPEAN PATENT APPLICATION**

(43) Date of publication:
**27.12.2017 Bulletin 2017/52**

(51) Int Cl.:
***A61B 3/16*** (2006.01)

(21) Application number: **17175287.6**

(22) Date of filing: **09.06.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **10.06.2016 IT UA20164269**

(71) Applicant: **Iromed Group S.r.l.**
**00144 Roma (RM) (IT)**

(72) Inventor: **CARUSO, Ciro**
**80128 Napoli (IT)**

(74) Representative: **Barbaro, Gaetano et al**
**Società Italiana Brevetti S.p.A.**
**Via G. Carducci, 8**
**20123 Milano (IT)**

(54) **APPLANATION HEAD FOR A GOLDMANN APPLANATION TONOMETER AND RELATED TONOMETER, METHOD FOR MEASURING AN INTRAOCULAR PRESSURE**

(57)    An applanation head for a Goldmann applanation tonometer having a pressure sensor mounted on the applanation head so as to measure at least one local pressure exerted on a portion of the applanation surface in contact with a flattened cornea, allows to measure accurately the intraocular pressure if an eye as well as the modulus of elasticity (Young's modulus) of the cornea.

Methods for calculating accurately the intraocular pressure and the modulus of elasticity in function of the local pressure exerted on a portion of the applanation surface in contact with a flattened cornea and of the overall pressure exerted by an eye pressed against the applanation surface of the Goldmann applanation tonometer are further disclosed.

**Fig. 7**

**Description**

TECHNICAL FIELD

This disclosure relates to tonometers for intraocular pressure measurements, and more particularly to a head for a Goldmann applanation tonometer, to a related Goldmann tonometer, to a method for measuring intraocular pressure of a human or animal eye and to a method for measuring the modulus of elasticity of a cornea.

BACKGROUND OF THE INVENTION

Intraocular pressure (IOP) measurement is a main task of standard ophthalmologic examinations, because an increase in its value is a major risk factor for glaucoma and is one of key parameters for diagnosing this disease, together with the evaluation of the optical papilla and with the visual field examination. Moreover, IOP measurement provides useful indications for diagnosing other ocular pathologies, such as uveitis, and for all patients undergoing to eye surgery, so as to be considered a routine examination to be performed in all ophthalmological visits.
Intraocular pressure is the result of a balance between production and outflow of aqueous humor and is influenced by numerous intrinsic and extrinsic factors (hereditary factors, race, axial length of the bulb, age, sex, systemic arterial pressure, body position, seasonal and nictemeral variations, physical activity, corneal thickness).
Intraocular pressure measurement may be performed by direct (manometry) or indirect (tonometry) methods. Ocular manometry is not used in clinical practice as being more invasive, but is limited to experimental use only. Instead, eye tonometry is the most commonly used method in clinical practice. It allows an indirect measurement of intraocular pressure by means of devices called tonometers, whose functioning principle is based on the relationship between intraocular pressure and force required to modify the natural shape of the cornea.
More precisely, tonometers do not measure intraocular pressure but ocular tension, which depends on both intraocular pressure and resistance that coating membranes oppose to deformation. Given that there is a relationship between tension and pressure, knowing the value of the former it is possible to measure the latter. In fact, in the present clinical language the terms eye pressure, intraocular pressure, ophthalmotone, ocular tension are considered equivalent to each other even if they indicate different concepts.
In the following, reference will be made only to applanation tonometers, which either measure the force needed to flatten a known corneal surface area or evaluate the amplitude of the flattened area by a fixed force, because the invention is applicable to them.
The term "applanation tonometer", in the following description and in the appended claims, refers to any tonometer using the principles of applanation tonometry to determine intraocular pressure.
Applanation tonometry is based on the fact that, to flatten a surface of area A of a cornea, a mean force F is required which acts on the area A so as to balance the intraocular pressure (IOP):

$$IOP = F/A$$

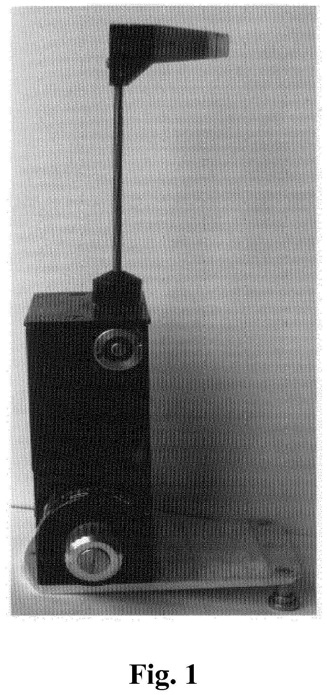

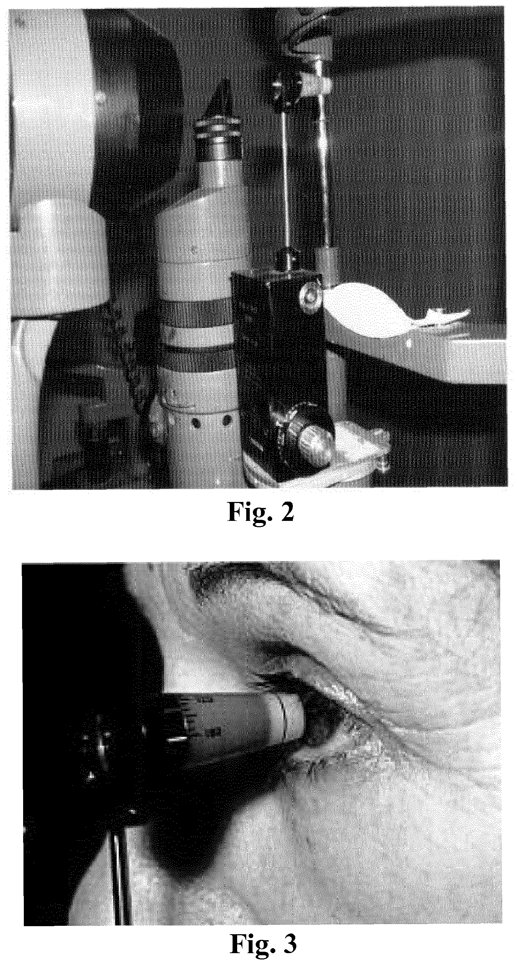

It follows that the pressure within an ideal sphere may be known by evaluating the width of the corneal area flattened by a known force or by evaluating the force needed to flatten a known corneal area. Therefore, two different types of tonometers can be used for applanation tonometry: variable area tonometers and variable force tonometers. The prototype of variable area tonometers is the Maklakov tonometer; after, the Halberg tonometer, the Poster-Inglina tonometer, the Barraquer tonometer, the Schiotz tonometer were derived, that are not widely used. The most known and used applanation tonometers in the world are variable force tonometers and undoubtedly among them the most famous is the Goldmann tonometer, now universally used and representing the international standard for IOP measurement.
A Goldmann tonometer, shown in figure 1, comprises a flattening element typically made of a transparent plastic head and containing a prism, joined by a rod to a spiral spring. Using a lateral graduated knob, it is possible to vary the value of the applied force on the cornea, which either is expressed in millimeters of mercury (mmHg) and is indicated by notches engraved on the knob or, in electronic tonometers, it is possible to be read the value of the IOP on a display.
The area of the flattened central corneal surface is 3,06 mm$^2$ and this value is fixed as a standard reference value.
The examination technique provides laying the tonometer on a suitable base of a slit lamp, shown in figure 2. After having applied aorneal anesthesia, a coloring dye is applied to the lower conjunctival cuff of the patient for coloring the ocular front surface, the dye being a fluorescent substance used to make even more evident the edge of the flattened area through observation under cobalt blue light of the slit lamp. As is well known, accurately identifying the edges of the flattened area is essential for proper eye pressure measurement.

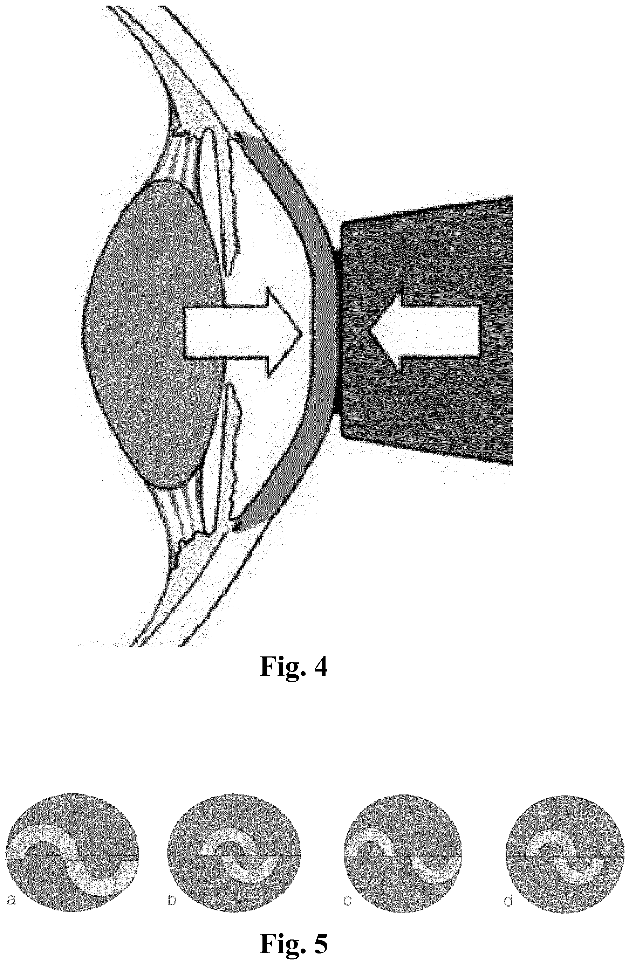

The patient is seated in front of the slit lamp and looks at a reference point as shown in figure 3. The slit of the lamp is opened at maximum and the blue cobalt filter is placed in order to better visualize the fluorescent ophthalmic dye, and this better highlights the edges of the flattened area. The cobalt blue light beam is then oriented so as to make it pass through the transparent prism and, independently on whether it is a manual tonometer or a digital tonometer, the knob is adjusted to the notch corresponding to 10 mmHg; for IOP measurement, it is possible to set the slit lamp forward so that the prism enters gently in contact with the central part of the cornea as shown in figure 4.

The prism, contained in the transparent plastic head, splits the circular image of the flattened corneal surface into two half-annuli, one close to the other. Through the eyepiece, two green half-circumferences in the bright blue cobalt background may be observed.

Figure 5 shows what is observed with the Goldmann tonometer in several cases. The lateral knob of the tonometer is rotated as far as the two half-circumferences are in contact with each other in correspondence of their inner edge. Depending on the exerted pressure, several aspects of the half-circumferences can be observed: if the half-circumferences are distant, it is necessary to increase the applanation force; if they cross over, you need to rotate the knob to reduce the exerted force. In figure 5A, the half-circumferences look relatively thick because of excessive use of ophthalmic dye; in figure 5B, the half-circumferences have an adequate thickness but are not properly aligned because the applanation force is excessive; in figure 5C, however, the applanation force is insufficient; in figure 5D, the half-circumferences are correctly aligned. At this point, the value of the IOP is determined by multiplying by ten the value on the knob.

Measurements carried out with the tonometer are influenced by the physical characteristics of the cornea, such as its thickness, its modulus of elasticity, as well as the attraction caused by the surface tension of the tear film, and in general it does not coincide with the intraocular pressure in the eye, measurable with a manometric technique by inserting a probe throughout the cornea. According to Goldmann, the inventor of the tonometer bearing his name, the effects of these physical characteristics would balance the attraction due to surface tension when a corneal surface of 3.06 mm diameter is flattened. Under such conditions, the pressure measured with a Goldmann tonometer would correspond to the intraocular pressure IOP.

Several scholars have found that this is not strictly accurate and various formulas have been proposed in the literature to correct measurement data provided by Goldmann tonometers in order to extrapolate the true IOP value. Some of these formulas do not take into account the corneal modulus of elasticity; other corrective formulas, very complex to use, are based on a standard hypothetical value of the corneal modulus of elasticity.

Unfortunately, the corneal modulus of elasticity of an elderly patient may be even twice than the corneal modulus of elasticity of a healthy young patient, so corrective formulas can introduce additional inaccuracies instead of improving accuracy.

The prior document US 2004/044278 discloses (par. [0001]) an applanation tonometer that includes an ultrasonic transducer measuring the corneal thickness in the applanation zone. Such a device would combine (par. [0023]) the pressure measurement in a traditional way with a pachymetric measurement performed by the ultrasonic sensor. The purpose is to measure a pressure value with a classical applanation tonometer, and then to correct the measured pressure value using correction formulas defined according to the corneal thickness measured by the ultrasonic sensor. The reliability of the device described in this prior document depends on the same correction formulas currently used for the Goldmann tonometer, thus it can lead to significant errors in the case of pressure measurements in elderly patients.

The prior document US 2005/0020896 discloses a tonometer, of the type currently sold under the trade name of TonoPen™, which is based on a different operating principle in respect to that of the Goldmann tonometer. In this tonometer, a pressure sensor (figure 2), installed in correspondence of a opaque flattening surface, is pressed against the corneal surface (Fig. 1) until all the surface of applanation is in contact with the cornea. In the described tonometer, an ultrasonic sensor (par. [0024]) is also installed to determine the corneal thickness, so as to adjust the value of the flattening pressure taking into account also the value of the corneal thickness (par. [0013]), with the same correction formulas used for the Goldmann tonometer.

SUMMARY OF THE INVENTION

Studies performed by the applicant have shown that the elastic reaction force exerted by a cornea flattened against the applanation surface of the head of a Goldmann tonometer is not uniformly distributed over the surface of applanation and depends upon the corneal modulus of elasticity, upon the central corneal thickness and upon the mean central radius of curvature of the cornea, i.e. the arithmetic mean between the inner central radius of curvature of the cornea and the outer central radius of curvature of the cornea.

An object of this disclosure is an applanation head for a Goldmann applanation tonometer as defined in the appended claim 1, as well as a related Goldmann applanation tonometer, having a pressure sensor mounted on the applanation head so as to measure at least one local pressure exerted on a portion of the applanation surface in contact with a flattened cornea. This portion of the applanation surface has a smaller area than the corneal surface that is flattened (according to the Goldmann standard, the flattened corneal surface must be circular and have a diameter of 3.06mm).

According to an embodiment, the pressure sensor installed on the applanation head is mounted so that its sensitive surface is coplanar with the applanation surface. According to yet another embodiment, the sensitive surface of the pressure sensor is circular and is concentric with the applanation surface.

According to a method of this disclosure, the value of the modulus of elasticity of a cornea is measured by measuring the pressure distribution or by measuring the local pressure only at an inner point of the applanation surface, then by extracting the value of the corneal modulus of elasticity through a function of such local pressure, of the overall pressure measured by Goldmann tonometer, and of estimated or measured values, of the central corneal thickness (CCT: Central Corneal Thickness), for example measured with any pachymeter, and of the mean central (apical) radius of curvature of the cornea, for example, measured by any keratometer.

According to another method of this disclosure, the intraocular pressure IOP of an eye is measured by correcting the overall pressure value measured by the Goldmann tonometer with a correction term that is dependent upon that local pressure and upon the pressure measured by the Goldmann tonometer.

These methods are implemented with a head according to this disclosure for a Goldmann tonometer and with a related Goldmann tonometer.

The claims as filed are an integral part of this description and are herein incorporated by reference.

BRIEF DESCRIPTION OF THE DRAWINGS

Figure 1 depicts a Goldmann tonometer.

Figure 2 depicts the Goldmann tonometer of figure 1 mounted on a base of a slit lamp.

Figure 3 shows the head of the Goldmann tonometer applied against the eye of a patient.

Figure 4 illustrates the principle of measurement of intraocular pressure with the Goldmann tonometer.

Figure 5 depicts half-circumferences or half-annuli displayed with the Goldmann tonometer, in the following cases: a) excessive use of ophthalmic dye; b) excessive applanation force; c) insufficient applanation force; d) correctly aligned half-circumferences.

Figure 6 depicts a head according to the present disclosure for a Goldmann tonometer, having a pressure sensor concentrical with the applanation surface.

Figure 7 depicts an applanation head for a Goldmann tonometer according to this disclosure, with an electric connector on a side surface for connecting the central pressure sensor with a microprocessor unit.

DETAILED DESCRIPTION

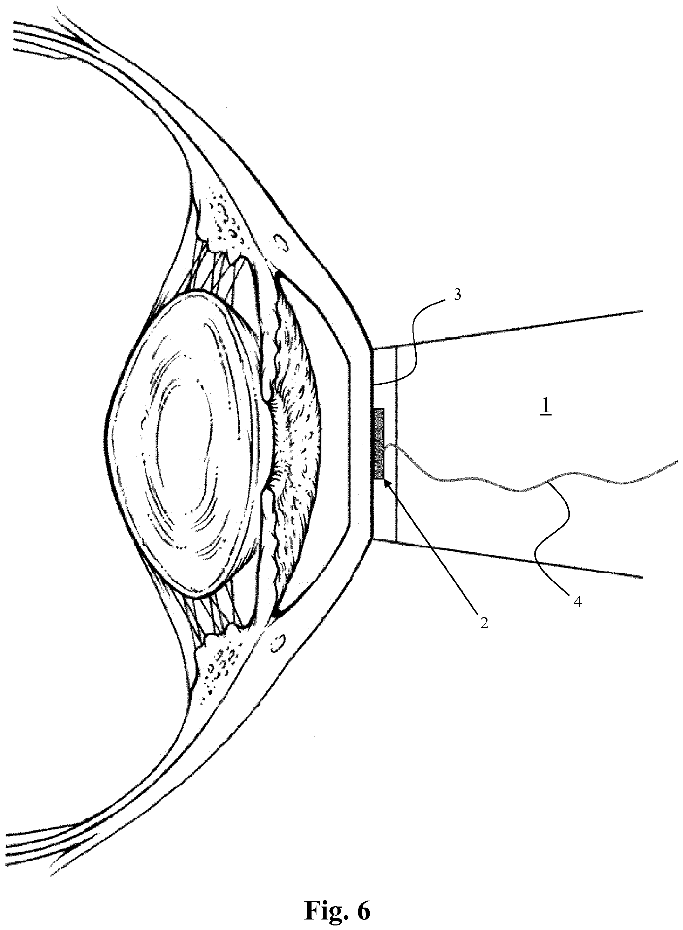

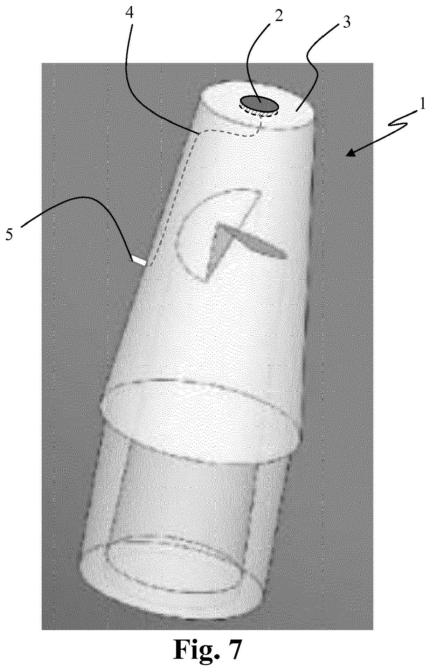

An exemplary embodiment of an applanation head (cone) 1 for a Goldmann tonometer according to this disclosure is schematically illustrated in figures 6 and 7. In the following, reference will be made to this embodiment, which is preferred, although different embodiments are possible, as will be apparent in the following.

Unlike the common applanation heads for Goldmann tonometers, the applanation head according to this disclosure has a pressure sensor 2 mounted on the applanation surface 3 intended to come into contact with the cornea, so that the sensitive surface of the pressure sensor 2 is coplanar with the transparent applanation surface of the tonometer head. All the optical parts of the tonometer applanation head (the "prisms" shown in transparency in figure 7) necessary to form the two half-circumferences of figure 5 by splitting the image of the circular perimeter of the surface of applanation of the head in contact with the flattened cornea, is identically replicated in the applanation head of this disclosure.

In the embodiment of figure 6, a connecting wire 4 is also shown, which is passed inside the tonometer head, for example in the space between the two prisms or alongside them (as in figure 7), and which protrudes either from the surface opposite to the applanation surface or from the lateral surface of the head (as in figure 7).

The remaining part of the tonometer according to this disclosure is not shown in detail because it is substantially similar to that of any Goldmann tonometer, except for the presence of a central unit, that will be further described hereinbelow. In fact, the tonometer head according to this disclosure may be mounted on the rod of any prior Goldmann tonometer, to transform it into a device suitable for measuring the intraocular pressure IOP and the corneal modulus of elasticity in a particularly accurate fashion. Although it may seem that the tonometer head of this disclosure looks like the part of the Schiotz tonometer that is resting upon the cornea, there are significant differences. Schiøtz's tonometer has a corneal support base that is shaped like a circular annulus and is curved and concave to better match with the corneal surface (approximately spherical). In the central hole of the support base (which is a concave circular annulus), a central piston slides against the cornea with a predetermined force, sinking slightly thereon; the more this central piston sinks into the cornea, the less intracellular pressure. A first difference between the tonometer head of this disclosure and the end of the Schiotz tonometer is that the base laying upon the cornea is not flat in the Schiotz tonometer but is curved and concave to better match the corneal curvature. A second difference is that the tip surface of the central piston that touches the cornea is not kept in the same plane as the support base that surrounds it: instead, the central piston has to sink into the cornea whilst the support base lies against the corneal surface, otherwise intraocular pressure cannot be measured.

Although globally the cornea cannot be assimilated to a spherical shell of constant thickness, the flattened portion during intraocular pressure measurement with a Goldmann tonometer is sufficiently small to be approximated with a spherical shell having a substantially constant thickness. Theoretical studies carried out by the applicant on the deformation of elastic spherical shells having a constant thickness, have shown that the distribution of pressure exerted by a flat cornea may be calculated using principles of elastic deformation theory and that it depends upon the central corneal thickness, upon the mean central radius of curvature of the cornea prior to applanation, upon the diameter of the flattened corneal surface, upon the pressure of the intraocular fluid (IOP) and upon the corneal modulus of elasticity. Such theoretical results have been confirmed by laboratory tests carried out by the applicant.

The central corneal thickness and the mean central corneal radius of curvature may be determined by means of a pachymetry and a keratometry, and given that in the Goldmann tonometer the diameter of the flattened corneal surface is predetermined (it is 3.06 mm) and it can be replicated very accurately thanks to the optical system (prisms, lenses) invented by Goldmann, then the distribution of pressure exerted by a flattened cornea against the transparent applanation surface can be expressed as a function of only two variables: the "true" intraocular pressure IOP and the corneal modulus of elasticity. Given that any known Goldmann tonometer detects the overall force exerted by a human eye with a flattened cornea against the tonometer head, it is sufficient to measure at least a value of a pressure locally exerted against a portion of the applanation surface in contact with a portion of the flattened cornea for extrapolating, through mathematical operations, the value of the "true" intraocular pressure IOP and the corneal modulus of elasticity.

A particularly convenient way to do this is shown in figures 6 and 7. The head 1 splits the image of the perimeter of the transparent applanation surface in contact with the cornea to display two half-circumferences (figure 5), thus it is advisable to install a pressure sensor 2 at the center of the applanation surface 3, that is at that part of it which is commonly called "blind zone" because it is not used to measure pressure with the method invented by Goldmann. According to a less preferred embodiment, the pressure sensor 2 can be installed at another part of the applanation surface 3 or even it may map the pressure distribution over the applanation surface.

Conveniently, the pressure sensor 2 will be a piezoelectric sensor with a circular sensitive surface of 1 mm diameter or less.

For example, it is possible to use a piezoelectric sensor of the same type of that used in the PASCAL™ tonometer (http://www.deviceoptical.com/pd_pascal.cfm#.V1bZtuSHM4Q).

In the PASCAL™ tonometer, such a sensor is installed behind a concave lens against which the corneal surface lays, so as to detect the force that a human eye exerts against the concave lens that, in theory, should have the same shape as a human cornea. Alternatively, it is possible to use also a different pressure sensor, such as for example the Murata SCB10H sensor, or the STMicroelectronics HLGA-10L sensor, or the Infineon DPS310 sensor.

In the following, reference will be made to the case in which the pressure sensor 2 has a circular sensitive surface. If this is not strictly true, the following formulas can be adapted using a radius "s" of an equivalent circle having the same area as the sensitive surface of the pressure sensor 2, still obtaining relatively accurate estimates.

Studies performed by the applicant showed that the average pressure exerted only by the elastic reaction of the flattened cornea against the head of the Goldmann tonometer is depends upon the central corneal thickness, upon the mean central radius of curvature of the cornea prior to applanation, upon the diameter of the flattened corneal surface, upon the intraocular fluid pressure (IOP) and upon the corneal modulus of elasticity. Without being bound to a theory, the applicant noted that this average pressure can be approximated as follows:

$$\frac{E \cdot h \cdot g^2}{R^3}$$

whrein

E = instantaneous local Young's module mediated over the corneal thickness;
h = central corneal thickness;
g = 1.53 mm is the radius of the applanated corneal surface with a Goldmann tonometer;
R = mean radius of curvature (inner radius plus outer radius, divided by 2) of the cornea measured at the apical portion, i.e. at the middle of the cornea.

The average pressure exerted only by the elastic reaction of the flattened cornea against the pressure sensor positioned at the center of the applanation surface may be approximated with the following function:

$$\frac{E \cdot h \cdot g^2}{R^3} \cdot \frac{s^2}{g^2} \cdot \left( 2 - \frac{s^2}{g^2} \right)$$

wherein:

s = radius of the circular pressure sensor placed at the center of the applanation surface. Conveniently, the circular pressure sensor placed at the center of the applanation surface will have a diameter of about 1 mm or less. With such a small size, the average pressure exerted only by the elastic reaction of the flattened cornea against the pressure sensor will be at least five times smaller than the average pressure exerted only by the elastic reaction of the flattened cornea against the head of the Goldmann tonometer.

The intraocular pressure IOP and the force of attraction due to the surface tension of the tear film are exerted against both the circular pressure sensor 2 and the entire applanation surface of 3 of the tonometer head, thus the difference between the average pressure $P_G$ measured by the Goldmann tonometer, measured by the knob on the tonometer rod, and the average pressure $P_S$ measured by the central circular sensor, will be independent of the intraocular pressure and the surface tension of the tear film and will be approximately given by:

$$P_G - P_S \cong \frac{E \cdot h \cdot g^2}{R^3} \cdot \left[ 1 - \frac{s^2}{g^2} \cdot \left( 2 - \frac{s^2}{g^2} \right) \right]$$

Knowing the central corneal thickness h by means of any corneal pachymeter and the mean central radius of curvature of the cornea R by means of any keratometer, knowing the radius s of the circular pressure sensor (it is a design data) and the radius g of the applanated surface with a Goldmann tonometer (it is always 1.53 mm), it is easy to calculate the corneal modulus of elasticity E:

$$E \cong \frac{P_G - P_S}{\dfrac{h \cdot g^2}{R^3} \cdot \left[ 1 - \dfrac{s^2}{g^2} \cdot \left( 2 - \dfrac{s^2}{g^2} \right) \right]}$$

Even in case the exact values of the central corneal thickness and of the mean central radius of curvature of the cornea are not known, and if one whishes to know only approximatively the value of the modulus of elasticity, it is possible to use, instead of the true value of "h" and of the true value of "R", even corresponding estimated values thereof, yet obtaining *in vivo* a reasonably accurate estimate of the value of the modulus of elasticity E. For example, the estimated values for "h" and for "R" may be respective nominal values of central corneal thickness $h_0$ and of (mean) radius of curvature $R_0$ of a standard cornea.

In practice, with the applanation head for a Goldmann tonometer of this disclosure, it is possible to measure the elastic response of a flattened cornea by measuring the pressure distribution or by measuring a local pressure in a portion (even at an inner point) of the applanation surface, then calculating the elastic reaction by combining these local pressure values $P_S$ and the overall pressure $P_G$ measured by the Goldmann tonometer. Differently from the prior tonometers disclosed in the documents US 2004/044278 and US 2005/0020896, according to this disclosure, the standard pressure measurement given by Goldmann tonometer is corrected using another pressure measurement (the local pressure).

Through another mathematical model, it is possible to correct the intraocular pressure $P_G$ measured by the Goldmann tonometer. Goldmann established standard conditions of central corneal thickness $h_0$, of (mean) radius of curvature $R_0$ and of modulus of elasticity (Young's modulus) $E_0$ for which, nominally, the pressure $P_G$ measured by the knob on the rod of its tonometer corresponded carefully to the intraocular pressure $P_{IOP}$. Commonly, it is believed that Goldmann tonometer measures practically exactly the intraocular pressure when it is equal to 15 mmHg (2kPa) and when the cornea has:

- a central corneal thickness equal to 0.536mm;

- a modulus of elasticity (Young's modulus) equal to 0.3MPa;
- an outer central radius of curvature of the cornea equal to 7.6 mm;
- an inner central radius of curvature of the cornea equal to 6.7 mm.

That is to say that:

Eo = 0.3MPa;
$h_0$ = 0.536 mm;
$R_0$ = 7.15 mm.

Given that the average pressure exerted only by the elastic reaction of the flattened cornea against the head of the Goldmann tonometer is roughly given by:

$$\frac{E \cdot h \cdot g^2}{R^3}$$

then it means that the pressure $P_G$ measured by the knob on the rod of the Goldmann tonometer is roughly given by:

$$P_G \cong P_{IOP} + \frac{E \cdot h \cdot g^2}{R^3} - \frac{E_0 \cdot h_0 \cdot g^2}{R_0^3}.$$

The "true" intraocular pressure $P_{IOP}$ can thus be obtained by the following formula:

$$P_{IOP} \cong P_G + \frac{E_0 \cdot h_0 \cdot g^2}{R_0^3} - \frac{P_G - P_S}{\left[1 - \frac{s^2}{g^2} \cdot \left(2 - \frac{s^2}{g^2}\right)\right]}$$

from the measurement of $P_G$ made by the Goldmann tonometer in the usual way, that is, when the cornea is flattened so that the two half-circumferences appear as in figure 5d, and from the average pressure $P_S$ detected by the central pressure sensor exactly when the two half-circumferences are as in figure 5d. It should be noticed that the previous formula does not depend upon the modulus of elasticity or upon the central corneal thickness, nor even by the mean central corneal radius of curvature. The radius "s" of the central pressure sensor (which is a design data), the values $E_0$, $h_0$, $R_0$ of a standard "cornea" according to Goldmann (which are nominal data available in literature), the radius g of the flattened corneal surface (fixed at 1.53 mm) are known, thus it is sufficient to measure the average pressure $P_S$ that the sensor measures in order to correct the effect of the corneal characteristics (thickness, rigidity, radius of curvature) on the pressure measurement $P_G$ carried out by the knob on the rod of the Goldmann tonometer. The Goldmann tonometer head of this disclosure may be mounted on the rod of any Goldmann tonometer to allow intraocular pressure measurement as usual and in addition to provide a measurement of the local pressure $P_S$ on the applanation surface.
This additional information, compared to prior Goldmann tonometers, allows to extrapolate the value of the modulus of elasticity of the cornea and to correct the pressure measurement $P_G$ made with the Goldmann tonometer. By combining the measurement of the pressure $P_G$ given by the Goldmann tonometer with the measurement of the pressure $P_S$ locally made by the sensor 2, it is possible to estimate the "true" intraocular pressure $P_{IOP}$ even if the central corneal thickness h, the modulus of elasticity E and the mean radius of curvature R of the cornea of the patient are unknown.
An advantage of the tonometer head of this disclosure is that it allows to estimate accurately the "true" intraocular pressure even if the modulus of elasticity varies during the measurement itself. It has been reported in the literature that the elasticity of the cornea is not constant during measurement with the Goldmann tonometer but tends to decline over time to settle at a base value and this causes a reduction in the pressure $P_G$ measured by the knob on the rod of the Goldmann tonometer. For this reason, it is commonly recommended to carry out quickly the measurement with the Goldmann tonometer. This is commonly explained with a decay of the corneal modulus of elasticity due to prolonged contact with the applanation surface.
This problem is overcome, or at least greatly attenuated, with the head for a Goldmann tonometer according to this disclosure, because the reduction of the corneal modulus of elasticity causes also a corresponding change of the pressure $P_S$ measured by the circular central pressure sensor. As a result, the difference between the pressures $P_G$-$P_S$ is reduced and the net result is that the difference

$$P_G - \frac{P_G - P_S}{\left[1 - \frac{s^2}{g^2} \cdot \left(2 - \frac{s^2}{g^2}\right)\right]}$$

is substantially unchanged, thus the equation

$$P_{IOP} \cong P_G + \frac{E_0 \cdot h_0 \cdot g^2}{R_0^3} - \frac{P_G - P_S}{\left[1 - \frac{s^2}{g^2} \cdot \left(2 - \frac{s^2}{g^2}\right)\right]}$$

will provide yet an accurate estimate of the "true" intraocular pressure of the patient because it is independent of the patient's corneal modulus of elasticity.

Conveniently, the Goldmann tonometer head of this disclosure will be mounted on the rod of a Goldmann tonometer of a known type. The connection wire of the central pressure sensor will end with an electrical connector 5 exiting from the lateral surface of the head 1, as shown in figure 7, in order to be connected with another electric wire fixed outside the tonometer rod to run parallel thereto.

Conveniently, a Goldmann tonometer equipped with the head of figure 6 will have also a central unit, preferably a microprocessor unit, that will receive the value of the pressure $P_S$ measured by the central sensor and the value of the pressure $P_G$ measured by the Goldmann tonometer, to generate the value of the "true" intraocular pressure with the previous formula. The electrical connection wire 4 of the pressure sensor 2, arranged along the lateral surface of the applanation head, will not shadow the contact point between the two half-circumferences of figure 5d because it will run transversely to the separation surface of the two internal prisms, so it will be possible to measure it with the Goldmann method without being disturbed by the presence of the wire 4.

Once an applanation head of the type shown in figure 7 is mounted on the rod of a Goldmann tonometer, it will be necessary to connect the electrical connector 5 that protrudes from the lateral surface of the applanation head 1 to an electrical wire leading to the microprocessor unit, in order to make the microprocessor read the pressure values measured by the pressure sensor.

The solution shown in figure 7 is particularly convenient for making disposable applanation head for Goldmann tonometers according to this disclosure. Indeed, applanation heads such as those shown in figure 7 will have only one additional component (the pressure sensor, likely a low cost sensor) and an electrical connector with respect to the common disposable applanation heads currently in use. The microprocessor unit, installed on a Goldmann tonometer according to this disclosure, should not be trown away as it can be disconnected from the disposable part (head) of the tonometer.

Conveniently, such a microprocessor unit will receive in digital form values of central corneal thickness and of mean central radius of curvature, manually entered by the operator or automatically entered by a pachymetric and keratometric measurement device functionally connected to the microprocessor unit, for calculating the corneal module of elasticity of the patient. In this way, it will be possible to study how the corneal modulus of elasticity varies with time when the cornea is flattened.

**Claims**

1. An applanation head for a Goldmann applanation tonometer, comprising:

a body having a flat and transparent applanation surface, said applanation surface being configured to be pressed against a cornea of an eye so as to flatten the cornea along a corneal surface having a pre-established area, said pre-established area being smaller than an area of said applanation surface of the body,
optical prisms contained in said body, configured to display in transparence in the form of two circular half-annuli a perimeter of said cornea surface flattened against the applanation surface,
**characterized in that**
said applanation head comprises a pressure sensor installed on said body and configured to measure at least a local pressure exerted against a portion of said applanation surface in contact with a flattened cornea, said portion of the applanation surface being smaller than said pre-established area of the flattened corneal surface.

2. The applanation head according to claim 1, wherein said pressure sensor:

- has a substantially flat sensing surface which is smaller than said applanation surface;
- is configured to generate at least an electrical signal when a pressure is exerted on the sensing surface;
- is installed onto said body so as said sensing surface is coplanar with said applanation surface.

3. The applanation head according to claim 2, wherein the sensing surface of said pressure sensor is substantially circular and is placed concentrically with said applanation surface.

4. The applanation head according to claim 3, wherein said pressure sensor has an electric connection wire placed inside said body and along a side surface of said body, and ending with an electric connector that protrudes from said body.

5. The applanation head according to claim 3 or 4, wherein said pressure sensor is a piezoelectric sensor having a diameter of 1mm or smaller.

6. A Goldmann applanation tonometer, comprising:

a supporting rod for an applanation head,
an applanation head, mounted on the supporting rod, having a flat and transparent applanation surface configured to be pressed against a cornea of an eye so as to flatten the cornea along a corneal surface having a pre-established area, said pre-established area being smaller than an area of said applanation surface of the body,
a force sensor connected to said supporting rod, configured for measuring an overall pressure exerted by an eye pressed against said applanation surface, **characterized in that**
said applanation head is of the type defined in one of claims from 1 to 5;
said tonometer comprises a central unit functionally connected to the pressure sensor installed onto the body of the applanation head in order to read a local pressure value, sensed by the pressure sensor, exerted against a portion of said applanation surface in contact with a flattened cornea, said portion of the applanation surface being smaller than said pre-established area of the flattened corneal surface.

7. The applanation tonometer according to claim 6, wherein said central unit is a microprocessor unit configured to:

- sense signals representing the local pressure value sensed by the pressure sensor,
- sense signals representing the overall pressure exerted by an eye pressed against said applanation surface,
- generate signals representing an intra-ocular pressure calculated upon said local pressure and upon said overall pressure.

8. The applanation tonometer according to claim 7, wherein:

said applanation head is of the type defined in claim 4 and is removably installed on the supporting rod;
said microprocessor unit is connected to the pressure sensor, installed onto the applanation head, through an electric wire that runs along the supporting rod and that is removably connected to the electric connector that protrudes from the body of the applanation head.

9. A method of measuring an intraocular pressure of an eye, comprising the following operations:

providing a Goldmann applanation tonometer according to one of claims from 6 to 8;
measuring, according to a Goldmann measurement method of an intraocular pressure, an overall pressure exerted by an eye pressed against the applanation surface of the Goldmann applanation tonometer and sensing at the same time a local pressure value, sensed by the pressure sensor installed onto the body of the applanation head, exerted against a portion of said applanation surface in contact with a flattened cornea;
calculating the intraocular pressure of the eye upon said local pressure and of said overall pressure.

10. A method of measuring an elasticity module of a cornea of an eye, being known of said cornea a central corneal thickness, a mean central radius of curvature obtained as an average between its inner central radius of curvature and its outer central radius of curvature, comprising the following operations:

providing a Goldmann applanation tonometer according to one of claims from 6 to 8;
sensing, according to a Goldmann measurement method of an intraocular pressure, an overall pressure exerted by an eye pressed against the applanation surface of the Goldmann applanation tonometer and sensing at the same time a local pressure value, sensed by the pressure sensor installed onto the applanation head, exerted against a portion of said applanation surface in contact with a flattened cornea;
calculating the elasticity module of the cornea upon said local pressure and upon said overall pressure.

Fig. 1

Fig. 2

Fig. 3

**Fig. 4**

**Fig. 5**

**Fig. 6**

Fig. 7

**REFERENCES CITED IN THE DESCRIPTION**

**Patent documents cited in the description**

- US 2004044278 A
- US 20050020896 A